# EUROPÄISCHE PATENTSCHRIFT

(11) **EP 2 211 832 B1**
(45) Veröffentlichungstag und Bekanntmachung des Hinweises auf die Patenterteilung: **29.03.2017**
(21) Anmeldenummer: 08836161.3
(22) Anmeldetag: 18.09.2008
(51) Int. Cl.: A61K 8/81, A61Q 5/06

(54) **HAARBEHANDLUNGSMITTEL, INSBESONDERE STYLINGMITTEL, ENTHALTEND ZWEI COPOLYMERE**
HAIR PREPARATION, PARTICULARLY STYLING AGENT, CONTAINING TWO COPOLYMERS
AGENTS DE TRAITEMENT CAPILLAIRE, NOTAMMENT PRODUITS COIFFANTS, CONTENANT DEUX COPOLYMÈRES

(30) Priorität: 04.10.2007 DE 102007047687
(43) Veröffentlichungstag der Anmeldung: 04.08.2010
(73) Patentinhaber: Henkel AG & Co. KGaA, 40589 Düsseldorf (DE)
(72) Erfinder: KNAPPE, Thorsten, 22869 Schenefeld (DE); SCHEFFLER, Rene, 25479 Ellerau (DE); BAYERSDÖRFER, Rolf, 22589 Hamburg (DE)
(86) Internationale Anmeldenummer: PCT/EP2008/062452
(87) Internationale Veröffentlichungsnummer: WO 2009/043732

(56) Entgegenhaltungen:
- WO-A1-98/19653
- WO-A1-99/13827
- US-B1- 6 375 932
- Datenblatt zu Luvimer® 100P, 36 D und 30 E XP007921915
- BASF: 'Luviflex® Soft. Film-forming agent for hair-styling mousses' INTERNET CITATION, [Online] Mai 2005, Seite 8PP, XP007908197 Gefunden im Internet: <URL:http://www.cosmetics.basf.de/pdf/State ments/Technical%20Informations/EN/Cosmetic% 20Ingredients/MEMKH%20100207e_Luviflex%20So ft.pdf> [gefunden am 2009-04-15]

## Beschreibung

Die Erfindung betrifft kosmetische Mittel, die zur Verformung keratinischer Fasern eingesetzt werden können, hergestellt durch Kombination von zwei unterschiedlichen Copolymeren vom Acrylat-Typ, die sich durch eine hohe Wasser- und Schweißbeständigkeit bei gleichzeitig guter Auswaschbarkeit auszeichnen.

Unter keratinischen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Der Einsatz von Polymeren in verschiedensten kosmetischen Mitteln ist weit verbreitet. Sie finden sich in Mitteln zur Behandlung der Haut ebenso wie in Mitteln zur Behandlung der Haare, in Mitteln, die unmittelbar nach der Anwendung wieder ab- bzw. ausgewaschen werden, so genannten Rinse-off Produkten, genauso wie in Mitteln, die auf Haut oder Haar verbleiben, so genannten Leave-on Mitteln. Dabei werden die Polymere aus unterschiedlichsten Gründen eingesetzt und jeweils bestimmte Eigenschaften der Polymere ausgenutzt. In Mitteln zur Hautbehandlung, in Shampoos, Haarspülungen und Haarkuren stehen oftmals die verdickenden oder pflegenden Eigenschaften der Polymere im Vordergrund. In Mitteln zur temporären Verformung keratinischer Fasern, im Folgenden auch Stylingmittel genannt, sind neben diesen Eigenschaften vor allem filmbildende und/oder festigende Wirkungen gefragt. Oftmals dienen Polymere auch als Hilfsmittel um die Abscheidung und Fixierung anderer Wirk- und Inhaltsstoffe auf der Haut oder dem Haar zu verbessern oder erst möglich zu machen. So lassen sich beispielsweise durch Zusatz geeigneter Polymere zu Haarfärbemitteln die Reibechtheiten und die Beständigkeit der Färbung erhöhen.

In der Regel enthalten kosmetische Mittel einzelne Polymere, die speziell darauf zugeschnitten sind, einen ganz bestimmten Effekt zu erzielen. Sollen verschiedene Effekte erzielt werden, ist die Zugabe mehrerer Polymere erforderlich. Werden allerdings zu viele verschiedene Polymere eingesetzt, kann das eine Reihe von Nachteilen mit sich bringen. So können Probleme bei der Formulierung entstehen, etwa weil die Polymere untereinander oder mit anderen Bestandteilen des Mittels reagieren und es zu Ausfällungen oder Zersetzungen kommt. Bestimmte Polymere neigen auch dazu, sich auf der Haut und insbesondere auf dem Haar so beständig abzuscheiden, dass sie bei einer gewöhnlichen Wäsche nicht mehr vollständig entfernt werden und es zu einer unerwünschten Anreicherung des Polymers und damit letztlich einer Belastung von Haut oder Haar kommt.

Es besteht daher ständig Bedarf an Polymeren oder geeigneten Kombinationen weniger Polymere, die möglichst viele der gewünschten Eigenschaften gleichzeitig aufweisen.

Beispielsweise ist es im Falle der Stylingmittel notwendig, dass die eingesetzten Polymeren dem behandelten Haar einen möglichst starken Halt geben. Neben einem hohen Haltegrad müssen Stylingmittel jedoch eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind neben niedriger Klebrigkeit und einem ausgewogenen Konditioniereffekt insbesondere die Beständigkeit des Haltes gegenüber Feuchtigkeit und Schweiß. Weiterhin soll ein Stylingmittel möglichst für alle Haartypen universell einsetzbar sein. Handelt es sich bei dem Stylingmittel um ein Gel oder eine Paste, sollen die Polymere zudem verdickende Eigenschaften besitzen. Schließlich muss sich das Stylingmittel nach Ende der Anwendung möglichst rückstandsfrei und einfach aus dem Haar durch herkömmliche Haarwäsche auswaschen lassen.

Aufgabe der vorliegenden Erfindung war es daher, geeignete Polymerkombinationen zur Verfügung zu stellen, die kosmetischen Mitteln bereits ohne Zusatz weiterer Wirkstoffe optimierte Eigenschaften verleihen. Insbesondere sollen die Polymerkombinationen verdickende und gleichzeitig filmbildende und/oder festigende Eigenschaften aufweisen. Stylingmittel, enthaltend die Polymere sollen einen sehr hohen Haltegrad aufweisen, ohne dass dabei auf Flexibilität und gute Feuchtebeständigkeit beispielsweise gegenüber Luftfeuchtigkeit, Regen sowie gegenüber Schweiß verzichtet werden müsste. Gleichzeitig muss eine gute Auswaschbarkeit der Stylingmittel durch herkömmliche Haarwäsche gewährleistet sein.

Es wurde nunmehr überraschenderweise gefunden, dass dies durch eine Kombination spezieller amphoterer Polymere erreicht werden kann.

Ein erster Anspruch der vorliegenden Erfindung ist daher ein kosmetisches Mittel, welches in einen kosmetisch akzeptablen Träger
a) mindestens ein Copolymer A gebildet aus
   - mindestens einem Monomer A1 ausgewählt aus Acrylsäure, Methacrylsäure, Acrylsäurealkylestern und Methacrylsäurealkylestern, und
   - mindestens einem amphoteren Monomer A2 ausgewählt aus (Meth)acryloylalkylbetainen der Formel A2-I 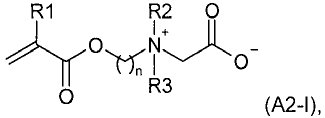 und (Meth)acryloylalkylaminoxiden der Formel A2-II 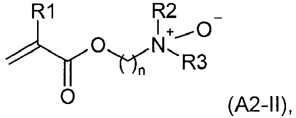 wobei in Formel A2-I und in Formel A2-II
      - R1 für H oder CH₃,
      - R2 und R3 jeweils unabhängig voneinander für gegebenenfalls verzweigtes C₁-C₁₀-Alkyl und
      - n für eine ganze Zahl von 1 bis 20 steht,
      und
b) mindestens ein von Copolymer A unterschiedliches Metharcylsäure/Ethylacrylat-Copolymer als filmbildendes und/oder festigendes anionisches Copolymer B enthält.

Filmbildende und/oder festigende amphotere Polymere B sind beispielsweise aus der im Jahr 2000 von der BASF veröffentlichten technischen Information zu Luvimer 100 P, Luvimer 36 D und Luvimer 36 E oder der im Jahr 2005 ebenfalls von der BASF veröffentlichen technischen Information zu Luviflex Soft bekannt. Gleiches gilt für Copolymere A und ihre Verwendung als filmbildende und/oder festigende Polymere, die beispielsweise in dem US-amerikanischen Patent US 6 375 932 B1 oder den internationalen Patentanmeldungen WO 98/19653 A1 und WO 99/13827 A1 beschrieben werden. Es hat sich nun überraschenderweise gezeigt, dass eine entsprechende Kombination beider Polymertypen selbstverdickende Eigenschaften besitzt, wobei die ausgezeichneten filmbildenden und/oder festigenden Eigenschaften der einzelnen Polymere noch erhöht werden. Stylingmittel, enthaltend eine Kombination dieser Polymere, zeichnen sich durch eine synergistische Erhöhung des Haltegrads und gute Feuchtebeständigkeit des erzielten Halts aus, ohne dabei in ihrer Auswaschbarkeit beeinträchtigt zu werden.

Als ersten zwingenden Bestandteil enthalten die erfindungsgemäßen kosmetischen Mittel mindestens ein Copolymer A.

Unter Copolymeren A, die aus den genannten Monomeren gebildet sind, werden im Sinne der vorliegenden Erfindung nur solche Copolymere verstanden, die neben Polymereinheiten, die aus dem Einbau der genannten Monomere M1 und M2 in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere A ausschließlich aus Polymereinheiten aufgebaut, die aus dem Einbau der genannten Monomere M1 und M2 in das Copolymer resultieren.

Bevorzugte Monomere A1 sind Acrylsäure, Methacrylsäure, Acrylsäure-C₁₋₂₀-alkylester und Methacrylsäure-C₁₋₂₀-alkylester.

Besonders bevorzugt ist Monomer A1 ausgewählt aus Acrylsäure, Methacrylsäure, Acrylsäuremethylester, Methacrylsäuremethylester, Acrylsäureethylester, Methacrylsäureethylester, Acrylsäurepropylester, Methacrylsäurepropylester, Acrylsäureisopropylester, Methacrylsäureisopropylester, Acrylsäureoctyllester, Methacrylsäureoctylester, Acrylsäuredecylester, Methacrylsäureccdecylester, Acrylsäurelauyrlester, Methacrylsäurelaurylester, Acrylsäuremyristylester, Methacrylsäuremyristylester, Acrylsäurecetylester, Methacrylsäurecetylester, Acrylsäurestearylester, Methacrylsäurestearylester, Acrylsäureeicosylester und Methacrylsäureeicosylester, ganz besonders bevorzugt aus Acrylsäure, Methacrylsäure, Acrylsäuremethylester, Methacrylsäuremethylester, Acrylsäureethylester, Methacrylsäureethylester, Acrylsäurelaurylester, Methacrylsäurelaurylester, Acrylsäurestearylester und Methacrylsäurestearylester.

Bevorzugte Monomere A2 sind (Meth)acryloylalkylbetaine der Formel A2-I und (Meth)acryloylalkylaminoxide der Formel A2-II, wobei R2 und R3 jeweils unabhängig voneinander für Methyl, Ethyl, *n-*Propyl, *i*-Propyl, *n*-Butyl, *i*-Butyl oder *t*-Butyl, besonders bevorzugt für Methyl stehen.

Bevorzugte Monomere A2 sind weiterhin ausgewählt aus (Meth)acryloylalkylbetainen der Formel A2-I und (Meth)acryloylalkylaminoxiden der Formel A2-II, wobei n jeweils für eine ganze Zahl von 1 bis 5, vorzugsweise für eine ganze Zahl von 1 bis 3 und besonders bevorzugt für 2 steht.

Bevorzugt sind auch Monomere A2 ausgewählt aus Methacryloylalkylbetainen der Formel A2-1 und Methacryloylalkylaminoxiden der Formel A2-II, wobei R1 jeweils für Methyl steht.

Ganz besonders bevorzugt ist ein Monomer A2 nach Formel A2-I oder A2-II, bei dem R1, R2 und R3 für Methyl und n für 2 stehen.

In einer ersten bevorzugten Ausführungsform enthält das erfindungsgemäße Mittel mindestens ein Copolymer A, das gebildet ist aus
- mindestens einem Monomer A1 ausgewählt aus Acrylsäure, Methacrylsäure, Acrylsäuremethylester, Methacrylsäuremethylester, Acrylsäureethylester, Methacrylsäureethylester, Acrylsäurepropylester, Methacrylsäurepropylester, Acrylsäureisopropylester und Methacrylsäureisopropylester, und
- aus einem Monomer A2 nach Formel A2-I, bei dem R1, R2 und R3 für Methyl und n für 2 stehen.

Entsprechende Copolymere (INCI-Bezeichnung: Methacryloyl ethyl betaine Acrylates Copolymer) sind bekannt und beispielsweise unter den Bezeichnungen Diaformer Z-400, Diaformer Z-AT, Diaformer Z-301 N, Diaformer Z-SM und Diaformer Z-W von der Firma Clariant, unter den Bezeichnungen Yukaformer 202, Yukaformer 204, Yukaformer 206 und Yukaformer 301 von der Firma Mitsubishi und unter den Bezeichnungen MIHAPOL^{®} PBS-50, MIHAPOL^{®} PBM-40 und MIHAPOL^{®} PBG-30 von der Firma Miwon Commercial Co., Ltd. erhältlich.

In einer zweiten bevorzugten Ausführungsform enthält das erfindungsgemäße Mittel mindestens ein Copolymer A, das gebildet ist aus
- mindestens zwei Monomeren A1,
   wobei das erste Monomer ausgewählt ist aus Acrylsäure, Methacrylsäure, Acrylsäuremethylester, Methacrylsäuremethylester, Acrylsäureethylester, und Methacrylsäureethylester,
   und das zweite Monomer ausgewählt ist aus Acrylsäurestearylester und Methacrylsäurestearylester, und
- aus einem Monomer A2 nach Formel A2-II, bei dem R1, R2 und R3 für Methyl und n für 2 stehen.

Diese Copolymere sind bekannt und beispielsweise unter der Bezeichnung Diaformer Z-632 von der Firma Clariant erhältlich, wobei der Einsatz von Diaformer Z-632 besonders bevorzugt ist.

In einer dritten bevorzugten Ausführungsform enthält das erfindungsgemäße Mittel mindestens ein Copolymer A, das gebildet ist aus
- mindestens drei Monomeren A1,
   wobei das erste Monomer ausgewählt ist aus Acrylsäure, Methacrylsäure, Acrylsäuremethylester, Methacrylsäuremethylester, Acrylsäureethylester, und Methacrylsäureethylester,
   das zweite Monomer ausgewählt ist aus Acrylsäurelaurylester und Methacrylsäurelaurylester,
   und das dritte Monomer ausgewählt ist aus Acrylsäurestearylester und Methacrylsäurestearylester, und
- aus einem Monomer A2 nach Formel A2-II bei dem R1, R2 und R3 für Methyl und n für 2 stehen.

Entsprechende Copolymere sind ebenfalls bekannt und beispielsweise unter den Bezeichnungen Diaformer^{®} Z-651 N, Diaformer^{®} Z-731 N, Diaformer^{®} Z-711 N, Diaformer^{®} Z-712N und Diaformer^{®} Z-712W von der Firma Clariant erhältlich, wobei der Einsatz von Diaformer Diaformer Z-651 N, Diaformer Z-731 N, Diaformer Z-711 N, Diaformer Z-712N und Diaformer Z-712W besonders bevorzugt ist.

Selbstverständlich ist es auch möglich, dass die erfindungsgemäßen Mittel eine Mischung aus mindestens zwei der Copolymere A enthalten, die gemäß der drei soeben beschriebenen bevorzugten Ausführungsformen eingesetzt werden.

Die erfindungsgemäßen Mittel enthalten das Copolymer A vorzugsweise in einer Menge von 0,01 bis 20 Gew.-%, bevorzugt 0,1 bis 15 Gew.-%, besonders bevorzugt 1,0 bis 12 Gew.-%, jeweils bezogen auf das gesamte Mittel.

Selbstverständlich können die erfindungsgemäßen Mittel auch mehrere Copolymere A enthalten, wobei die Gesamtmenge an Copolymer A vorzugsweise jedoch bei maximal 20 Gew.-% liegt.

Die Copolymere A lassen sich mittels der bekannten Polymerisationsmethoden aus den genannten Monomeren herstellen und sind in der Regel kommerziell verfügbar.

Als zweiten zwingenden Bestandteil enthalten die erfindungsgemäßen Mittel zur temporären Verformung keratinischer Fasern mindestens ein von Copolymer A verschiedenes filmbildendes und/oder festigendes anionisches Copolymer B.

Das erfindungsgemäße Mittel enthält als filmbildendes und/oder festigendes anionisches Copolymer B ein Methacrylsäure/Ethylacrylat-Copolymer, insbesondere bevorzugt das Copolymer, das von der Firma BASF AG unter der Bezeichnung Luviflex^{®} Soft (INCI-Bezeichnung: Acrylates Copolymer) vertrieben wird.

Das filmbildende und/oder festigende anionisches Copolymer B ist vorzugsweise in einer Menge von 0,01 bis 20 Gew.-%, vorzugsweise 0,1 bis 15 Gew.-%, besonders bevorzugt 1,0 bis 10 Gew.-%, bezogen auf das gesamte Mittel, enthalten. Selbstverständlich können auch mehrere filmbildende und/oder festigende anionische Copolymere B und/oder B' enthalten sein, wobei die Gesamtmenge an filmbildenden und/oder festigenden anionischen Copolymeren B und B' vorzugsweise jedoch bei maximal 20 Gew.-% liegt.

Um die gewünschten Eigenschaften des erfindungsgemäßen Mittels zu erzielen, muss das Mittel sowohl Copolymer A, als auch ein von Copolymer A verschiedenes filmbildendes und/oder festigendes anionisches Copolymer B enthalten. Insbesondere die für Stylingmittel wünschenswerte Kombination aus sehr starkem Halt und ausgezeichneter Feuchtebeständigkeit bei gleichzeitiger guter Auswaschbarkeit lassen sich so erhalten. Es hat sich gezeigt, dass sich ein optimales Eigenschaftsprofil ergibt, wenn das Mittel das Copolymer A und das filmbildende und/oder festigende anionische Copolymer B in einem Gewichtsverhältnis von 1:20 bis 20:1, vorzugsweise von 1:10 bis 10:1, besonders bevorzugt von 1:5 bis 5:1, ganz besonders bevorzugt 1:2 bis 2:1 enthält.

Neben Copolymer A und filmbildenden und/oder festigenden anionischen Polymeren B können die Mittel weiterhin alle weiteren bekannten filmbildenden und/oder festigenden Polymere enthalten. Dabei können diese filmbildenden und/oder festigenden Polymere sowohl permanent als auch temporär kationisch, anionisch, amphoter oder nichtionisch sein.

Da Polymere häufig multifunktional sind, können deren Funktionen nicht immer klar und eindeutig voneinander abgegrenzt werden. Insbesondere gilt dies für filmbildende und festigende Polymere. Es wird an dieser Stelle jedoch explizit darauf verwiesen, dass im Rahmen der vorliegenden Erfindung sowohl filmbildende als auch festigende Polymere wesentlich sind. Da beide Eigenschaften auch nicht völlig unabhängig voneinander sind, werden unter dem Begriff "festigende Polymere" auch immer "filmbildende Polymere" verstanden und umgekehrt.

Zu den bevorzugten Eigenschaften der filmbildenden Polymeren zählt die Filmbildung. Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Alkohol oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

Geeignete weitere synthetische, filmbildende, haarfestigende Polymere sind beispielsweise Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C₁- bis C₇-Alkylgruppen, besonders bevorzugt C₁- bis C₃-Alkylgruppen sind.

Beispielhaft seien genannt Homopolymere des Vinylcaprolactams, des Vinylpyrrolidons oder des N-Vinylformamids. Weitere geeignete synthetische, filmbildende, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akypomine^{®} P 191 von der Firma CHEM-Y, Emmerich, oder Sepigel^{®} 305 von der Firma Seppic vertrieben werden; Polyvinylalkohole, die beispielsweise unter den Handelsbezeichnungen Elvanol^{®} von Du Pont oder Vinol^{®} 523/540 von der Firma Air Products vertrieben werden sowie Polyethylenglykol/Polypropylenglykol-Copolymere, die beispielsweise, unter den Handelsbezeichnungen Ucon^{®} der Union Carbide vertrieben werden.

Besonders erfindungsgemäß bevorzugte, filmbildende und/oder festigende Copolymere werden beispielsweise unter der Handelsbezeichnung Luviquat^{®} Supreme (INCI-Bezeichnung: Polyquaterium-68) von der Firma BASF AG, Ludwigshafen, unter der Bezeichnung Acudyne^{®} LT-120 (INCI-Bezeichnung: Acrylates/C1-2 Succinates/Hydroxyacrylates Copolymer) von der Firma Rohm and Haas, Personal Care, und unter der Bezeichnung Amphomer^{®} (INCI-Bezeichnung: Octylacrylamide / Acrylates / Butylaminoethyl Methacrylate Copolymer) von der Firma National Starch vertrieben. Geeignete natürliche filmbildende Polymere sind z.B. Cellulosederivate, z. B. Hydroxypropylcellulose mit einem Molekulargewicht von 30.000 bis 50.000 g/mol, welche beispielsweise unter der Handelsbezeichnung Nisso SI^{®} von der Firma Lehmann & Voss, Hamburg, vertrieben wird. Festigende Polymere tragen zum Halt und/oder zum Aufbau des Haarvolumens und der Haarfülle der Gesamtfrisur bei. Diese so genannten festigenden Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Substanzen, welche dem Haar weiterhin hydrophobe Eigenschaften verleihen, sind hierbei bevorzugt, weil sie die Tendenz des Haares, Feuchtigkeit, also Wasser, zu absorbieren, verringern. Dadurch wird das schlaffe Herunterhängen der Haarsträhnen vermindert und somit ein lang anhaltender Frisurenaufbau und -erhalt gewährleistet. Als Testmethode hierfür wird häufig der so genannte curl-retention - Test angewendet. Diese polymeren Substanzen können weiterhin erfolgreich in leave-on und rinse-off Haarkuren oder Shampoos eingearbeitet werden. Da Polymere häufig multifunktional sind, das heißt mehrere anwendungstechnisch erwünschte Wirkungen zeigen, finden sich zahlreiche Polymere in mehreren auf die Wirkungsweise eingeteilten Gruppen, so auch im CTFA Handbuch. Sofern die erfindungsgemäßen Mittel weitere filmbildende und/oder festigende Polymere enthalten, werden diese vorzugsweise in einer Menge von 0,01 bis 20 Gew.-%, vorzugsweise 0,1 bis 15 Gew.-%, bezogen auf das gesamte Haarfestigungsmittel, eingesetzt. Selbstverständlich können auch mehrere filmbildende und/oder festigende Polymere enthalten sein, wobei die Gesamtmenge an weiteren filmbildenden und/oder festigenden Polymeren vorzugsweise jedoch bei maximal 20 Gew.-% liegt.

In einer bevorzugten Ausführungsform enthalten die erfindungsgemäßen Mittel als filmbildende und/oder festigende Polymere ausschließlich Copolymere A und filmbildende und/oder festigende anionische Polymere B.

Die erfindungsgemäßen Mittel enthalten die Polymere in einem kosmetisch akzeptablen Träger. Bevorzugte kosmetisch akzeptable Träger sind wässrige, alkoholische oder wässrigalkoholische Medien mit vorzugsweise mindestens 10 Gew.-% Wasser, bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gew.-% bezogen auf das gesamte Mittel.

Die Mittel weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C, sofern nichts anderes vermerkt ist.

Die erfindungsgemäßen Mittel können weiterhin die Hilfs- und Zusatzstoffe enthalten, die üblicherweise den jeweiligen kosmetischen Mitteln zugesetzt werden.

Als geeignete Hilfs- und Zusatzstoffe sind insbesondere Pflegestoffe zu nennen. Diese finden sowohl bei Haut- als auch Haarbehandlungsmitteln Anwendung und können bei geeigneter Wahl des Pflegestoffs beispielsweise in Cremes, Shampoos, Haarspülungen, Haarkuren, Gele, Pump- und Aerosolsprays und Schaumprodukte eingearbeitet werden.

Als Pflegestoff kann beispielsweise ein Silikonöl und/oder ein Silikongum eingesetzt werden. In einer besonderen Ausführungsform der Erfindung enthalten die Mittel mindestens ein Silikonöl und/oder ein Silikongum.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Silikonöle bewirken die unterschiedlichsten Effekte. So beeinflussen sie beispielsweise gleichzeitig die Trocken- und Nasskämmbarkeiten, den Griff des trockenen und nassen Haares sowie den Glanz. Unter dem Begriff Silikonöle versteht der Fachmann mehrere Strukturen Silicium-organischer Verbindungen. Zunächst werden hierunter die Dimethiconole (S1) verstanden. Diese können sowohl linear als auch verzweigt als auch cyclisch oder cyclisch und verzweigt sein.

Dimethicone (S2) bilden die zweite Gruppe der Silikone, welche erfindungsgemäß enthalten sein können. Diese können sowohl linear als auch verzweigt als auch cyclisch oder cyclisch und verzweigt sein.

Dimethiconcopolyole (S3) bilden eine weitere Gruppe von Silikonen, die geeignet sind. Entsprechende Dimethiconcopolyole sind kommerziell erhältlich und werden beispielsweise von der Firma Dow Corning unter der Bezeichnung Dow Corning ^{®} 5330 Fluid vertrieben.

Selbstverständlich umfasst die erfindungsgemäße Lehre auch, dass die Dimethiconole, Dimethicone und/oder Dimethiconcopolymere bereits als Emulsion vorliegen können. Dabei kann die entsprechende Emulsion der Dimethiconole, Dimethicone und/oder Dimethiconcopolyole sowohl nach der Herstellung der entsprechenden Dimethiconole, Dimethicone und/oder Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole, Dimethicone und/oder Dimethiconcopolyole auch direkt durch ein Emulsionspolymerisationsverfahren hergestellt werden. Auch derartige Verfahren sind dem Fachmann wohl bekannt.

Wenn die Dimethiconole, Dimethicone und/oder Dimethiconcopolyole als Emulsion verwendet werden, dann beträgt die Tröpfchengröße der emulgierten Teilchen erfindungsgemäß 0,01 bis 10000 µm, bevorzugt 0,01 bis 100 µm, besonders bevorzugt 0,01 bis 20 µm und ganz besonders bevorzugt 0,01 bis 10 µm. Die Teilchengröße wird dabei nach der Methode der Lichtstreuung bestimmt.

Werden verzweigte Dimethiconole, Dimethicone und/oder Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Erfindung ist daher unter verzweigten Dimethiconolen, Dimethiconen und/oder Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole, Dimethicone und/oder Dimethiconcopolyole ganz besonders bevorzugt sein.

Geeignete Silikone sind weiterhin aminofunktionelle Silikone (S4), insbesondere die Silikone, die unter der INCI-Bezeichnung Amodimethicone zusammengefasst sind. Darunter sind Silikone zu verstehen, welche mindestens eine, gegebenenfalls substituierte, Aminogruppe aufweisen.

Vorzugsweise werden solche aminofunktionellen Silikone eingesetzt, die eine Aminzahl oberhalb von 0,25 meq/g, vorzugsweise oberhalb von 0,3 meq/g und insbesondere bevorzugt oberhalb von 0,4 meq/g aufweisen. Die Aminzahl steht dabei für die Milli-Äquivalente Amin pro Gramm des aminofunktionellen Silikons. Sie kann durch Titration ermittelt und auch in der Einheit mg KOH/g angegeben werden.

Weitere geeignete Silikone sind beispielsweise
- oligomere Polydimethylcyclosiloxane (INCI-Bezeichnung: Cyclomethicone), insbesondere die tetramere und die pentamere Verbindung, die als Handelsprodukte DC 245 Fluid, DC 344 bzw. DC 345 von Dow Corning vertrieben werden,
- Hexamethyl-Disiloxan (INCI-Bezeichnung: Hexamethyldisiloxane), z. B. das unter der Bezeichnung Abil^{®} K 520 vertriebenen Produkt,
- Polyphenylmethylsiloxane (INCI-Bezeichnung: Phenyl Trimethicone), z. B. das Handelsprodukt DC 556 Cosmetic Grade Fluid von Dow Corning,
- Ester sowie Partialester der Silikon-Glykol-Copolymere, wie sie beispielsweise von der Firma Fanning unter der Handelsbezeichnung Fancorsil^{®} LIM (INCI-Bezeichnung: Dimethicone Copolyol Meadowfoamate) vertrieben werden,
- anionische Silikonöle, wie beispielsweise das Produkt Dow Corning^{®}1784.

Gemäß einer bevorzugten Ausführungsform enthält das erfindungsgemäße Mittel mindestens zwei unterschiedliche Silikonderivate, insbesondere bevorzugt eine Kombination aus einem flüchtigen und einem nichtflüchtigen Silikon. Flüchtig im Sinne der Erfindung sind solche Silikone, die eine Flüchtigkeit aufweisen, die gleich oder größer als die Flüchtigkeit des cyclischen, pentameren Dimethylsiloxans ist. Solche Kombinationen sind auch als Handelsprodukte (z. B. Dow Corning^{®}1401, Dow Corning^{®}1403 und Dow Corning^{®}1501, jeweils Mischungen aus einem Cyclomethicone und einem Dimethiconol) erhältlich.

Bevorzugte Mischungen verschiedener Silikone sind beispielsweise Dimethicone und Dimethiconole, lineare Dimethicone und cyclische Dimethiconole. Eine ganz besonders bevorzugte Mischung von Silikonen besteht aus mindestens einem cyclischen Dimethiconol und/oder Dimethicon, mindestens einem weiteren nicht cyclischen Dimethicon und/oder Dimethiconol sowie mindestens einem aminofunktionellen Silikon.

Werden unterschiedliche Silikone als Mischung verwendet, so ist das Mischungsverhältnis weitgehend variabel. Bevorzugt werden jedoch alle zur Mischung verwendeten Silikone in einem Verhältnis von 5 : 1 bis 1 : 5 im Falle einer binären Mischung eingesetzt. Ein Verhältnis von 3 : 1 bis 1 : 3 ist besonders bevorzugt. Ganz besonders bevorzugte Mischungen enthalten alle in der Mischung enthaltenen Silikone weitestgehend in einem Verhältnis von etwa 1 : 1, jeweils bezogen auf die eingesetzten Mengen in Gew.-%.

Die Mittel enthalten die Silikone bevorzugt in Mengen von 1-25 Gew.-%, besonders bevorzugt von 5 - 20 Gew.-% und insbesondere bevorzugt von 7-15 Gew.-%, bezogen auf das gesamte Mittel.

Obwohl das erfindungsgemäße Mittel als Pflegestoff vorzugsweise ein Silikonderivat enthält, ist es auch möglich, dass das Mittel statt oder neben einer Silikonkomponente mindestens einen Pflegestoff einer anderen Verbindungsklasse enthält.

Als Pflegestoff einer anderen Verbindungsklasse kann das Mittel beispielsweise mindestens ein Proteinhydrolysat und/oder eines seiner Derivate enthalten.

Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff einer anderen Verbindungsklasse sind weiterhin kationische Tenside geeignet.

Erfindungsgemäß bevorzugt sind kationische Tenside vom Typ der quartären Ammoniumverbindungen, der Esterquats und der Amidoamine. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäßen Mitteln bevorzugt in Mengen von 0,05 bis 10 Gew.-%, bezogen auf die gesamte Anwendungszubereitung, enthalten. Mengen von 0,1 bis 5 Gew.-% sind besonders bevorzugt.

Als Pflegestoff eignen sich ebenfalls pflegende Polymere. Es sei an dieser Stelle darauf hingewiesen, dass einige pflegende Polymere auch filmbildende und/oder festigende Eigenschaften aufweisen, und daher auch bei der Aufzählung geeigneter filmbildender und/oder festigender Polymere genannt sein können.

Eine erste Gruppe der pflegenden Polymere sind die kationischen Polymere. Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I), 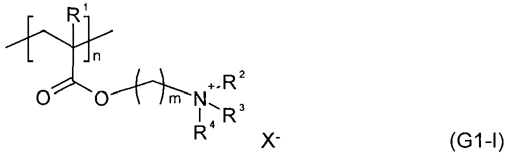
in der R¹= -H oder -CH₃ ist, R², R³ und R⁴ unabhängig voneinander ausgewählt sind aus C₁₋₄-Alkyl-,-Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt: R¹ steht für eine Methylgruppe
R², R³ und R⁴ stehen für Methylgruppen
m hat den Wert 2.

Als physiologisch verträgliche Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C-₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quartären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silikon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und - methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®} ASCP 1011, Gafquat^{®}HS 110, Luviquat^{®}8155 und Luviquat^{®} MS 370 erhältlich sind.

Weitere erfindungsgemäß einsetzbare kationische Polymere sind die so genannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugt eingesetzte kationische Polymere sind kationische Cellulose-Derivate und Chitosan und dessen Derivate, insbesondere die Handelsprodukte Polymer^{®}JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Weiterhin sind kationisierte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder eine Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen.

Ganz besonders bevorzugt sind die kationischen Proteinhydrolysate und -derivate auf pflanzlicher Basis.

Bevorzugt eingesetzte amphotere Polymere sind solche Polymerisate, die sich im wesentlichen zusammensetzen aus
(a) Monomeren mit quartären Ammoniumgruppen der allgemeinen Formel (II),

   R¹-CH=CR²-CO-Z-(CₙH₂ₙ)-N⁽⁺⁾R³R⁴R⁵ A⁽⁻⁾ (II)

   in der R¹ und R² unabhängig voneinander stehen für Wasserstoff oder eine Methylgruppe und R³, R⁴ und R⁵ jeweils unabhängig voneinander für eine Alkylgruppe mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (III),

   R⁶-CH=CR⁷-COOH (III)

   in denen R⁶ und R⁷ unabhängig voneinander für Wasserstoff oder eine Methylgruppe stehen.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyltrimethyl-ammoniumchlorid ist ein besonders bevorzugtes Monomeres (a). Als Monomeres (b) für die genannten Polymerisate wird bevorzugt Acrylsäure verwendet.

Die erfindungsgemäßen Mittel enthalten die pflegenden, kationischen und/oder amphoteren Polymere in bevorzugter Weise in einer Menge von 0,01 bis 5 Gew.-%, insbesondere in einer Menge von 0,1 bis 2 Gew.-%, jeweils bezogen auf die gesamte Anwendungszubereitung.

Als Pflegestoff kann das erfindungsgemäße Mittel weiterhin mindestens ein Vitamin, ein Provitamin, eine Vitaminvorstufe und/oder eines derer Derivate enthalten.

Dabei sind erfindungsgemäß solche Vitamine, Provitamine und Vitaminvorstufen bevorzugt, die üblicherweise den Gruppen A, B, C, E, F und H zugeordnet werden.

Bevorzugt enthalten die erfindungsgemäßen Mittel Vitamine, Provitamine und Vitaminvorstufen aus den Gruppen A, B, C, E und H.

Panthenol, Pantolacton, Pyridoxin und seine Derivate sowie Nicotinsäureamid und Biotin sind besonders bevorzugt.

Ganz besonders bevorzugt wird als Pflegestoff D-Panthenol, gegebenenfalls in Kombination mit mindestens einem der oben genannten Silikonderivate eingesetzt.

Als Pflegestoff können die erfindungsgemäßen Mittel weiterhin mindestens einen Pflanzenextrakt enthalten.

Als Pflegestoff eignet sich weiterhin eine Reihe von Carbonsäuren. Vorteilhaft im Sinne der Erfindung können insbesondere kurzkettige Carbonsäuren sein. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettige oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Weiterhin ist es erfindungsgemäß bevorzugt 2-Pyrrolidinon-5-carbonsäure und deren Derivate als Carbonsäure einzusetzen. Besonders bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen vorzugsweise 0,05 bis 10 Gew.%, bezogen auf die gesamte Anwendungszubereitung, besonders bevorzugt 0,1 bis 5 Gew.%, und insbesondere bevorzugt 0,1 bis 3 Gew.%.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren einzusetzen. Hierbei hat sich gezeigt, dass neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeigneten Hydroxycarbonsäureester sind Ester der ß-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8-22 C-Atomen, also z.B. Fettalkohole oder synthetische Fettalkohole. Dabei sind die Ester von C₁₂-C₁₅-Fettalkoholen besonders bevorzugt. Ester dieses Typs sind im Handel erhältlich, z.B. unter dem Warenzeichen Cosmacol® der EniChem, Augusta Industriale. Besonders bevorzugte Polyhydroxypolycarbonsäuren sind Polymilchsäure und Polyweinsäure sowie deren Ester.

Als Pflegestoff eignen sich weiterhin Ectoin oder Ectoinderivate, Allantoin, Taurin und/oder Bisabolol.

Die erfindungsgemäßen Mittel enthalten diese Pflegestoffe bevorzugt in Mengen von 0,001 bis 2, insbesondere von 0,01 bis 0,5 Gew.-%, jeweils bezogen auf die gesamte Anwendungszubereitung.

Auch Mono- bzw. Oligosaccharide können als Pflegestoff in den erfindungsgemäßen Mitteln eingesetzt werden.

Es können sowohl Monosaccharide als auch Oligosaccharide, wie beispielsweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.

Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist.

Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit. Bevorzugte Glykoside sind die Methylglucoside.

Die Mono- bzw. Oligosaccharide sind in den erfindungsgemäßen Mitteln bevorzugt in einer Menge von 0,1 bis 8 Gew.-%, insbesondere bevorzugt 1 bis 5 Gew.-%, bezogen auf die gesamte Anwendungszubereitung, enthalten.

Das Mittel kann weiterhin mindestens ein Lipid als Pflegestoff enthalten.

Erfindungsgemäß geeignete Lipide sind Phospholipide, beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona, unter den Handelsbezeichnungen Phospholipid EFA^{®}, Phospholipid PTC^{®} sowie Phospholipid SV^{®} vertrieben.

Die erfindungsgemäßen Mittel enthalten die Lipide bevorzugt in Mengen von 0,01 - 10 Gew.-%, insbesondere 0,1 - 5 Gew.-%, bezogen auf die gesamte Anwendungszubereitung.

Weiterhin sind als Pflegestoff Ölkörper geeignet.

Zu den natürlichen und synthetischen kosmetischen Ölkörpern sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyln-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein.

Die Einsatzmenge der natürlichen und synthetischen kosmetischen Ölkörper in den erfindungsgemäßen Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Obwohl jeder der genannten Pflegestoffe für sich alleine bereits ein zufrieden stellendes Resultat ergibt, sind im Rahmen der vorliegenden Erfindung auch alle Ausführungsformen umfasst, in denen das Mittel mehrere Pflegestoffe auch aus verschiedenen Gruppen enthält.

Durch Zugabe eines UV-Filters können sowohl die Mittel selbst, als auch die behandelte Haut oder die Haare vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In Abhängigkeit von der Art des erfindungsgemäßen Mittels kann es erforderlich sein, dass diese weiterhin mindestens ein Tensid enthalten. Dies gilt insbesondere für Hautreinigungsmittel und Shampoos. Aber auch andere Mittel, wie beispielsweise Haarspülungen, Haarkuren und bestimmte Stylingmittel, insbesondere Stylingschäume, können Tenside enthalten.

Beispielsweise können kationische Tenside eingesetzt werden, wie sie bereits oben als geeignete Pflegestoffe beschrieben sind. Bezüglich der bevorzugten kationischen Tenside und der eingesetzten Mengen gelten obige Ausführungen entsprechend.

Neben oder statt der kationischen Tenside können die Mittel weitere Tenside oder Emulgatoren enthalten, wobei prinzipiell sowohl anionische als auch ampholytische und nichtionische Tenside und alle Arten bekannter Emulgatoren geeignet sind. Die Gruppe der ampholytischen oder auch amphoteren Tenside umfasst zwitterionische Tenside und Ampholyte. Die Tenside können bereits emulgierende Wirkung haben.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓCH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CHzO)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I), 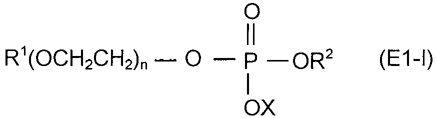 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C1 bis C4 - Kohlenwasserstoffrest, steht,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906 beschrieben sind,
- Monoglyceridsulfate und Monoglyceridethersulfate der Formel (E1-III) 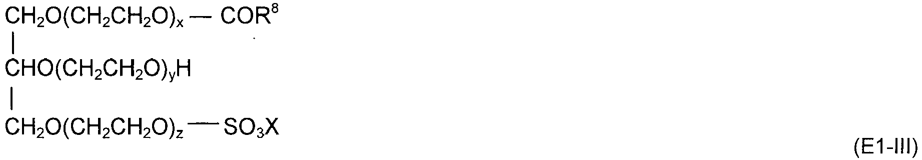 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer C₈-C₂₄ - Alkyl- oder -Acylgruppe im Molekül mindestens eine freie Aminogruppe und mindestens eine - COOH- oder -SO₃H-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das C₁₂ - C₁₈ - Acylsarcosin.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin können als nichtionische Tenside die Zuckertenside enthalten sein. Diese sind bevorzugt in Mengen von 0,1 bis 20 Gew.-%, bezogen auf die jeweilige gesamte Zusammensetzung, enthalten. Mengen von 0,5 bis 15 Gew.-% sind besonders bevorzugt, und ganz besonders bevorzugt sind Mengen von 0,5 bis 7,5 Gew.-%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die weiteren Tenside werden in der Regel in Mengen von 0,1 bis 45 Gew.-%, bevorzugt 0,5 bis 30 Gew.-% und ganz besonders bevorzugt von 0,5 bis 25 Gew.-%, bezogen auf die jeweilige gesamte Zusammensetzung, eingesetzt. Dabei hängt die eingesetzte Menge wesentlich davon ab, welchen Zweck das erfindungsgemäße Mittel erfüllt. Handelt es sich um ein Shampoo oder ein anderes reinigendes Mittel, sind auch Tensidmengen über 45 Gew.-% üblich.

Die Mittel können weiterhin mindestens einen Emulgator enthalten. Erfindungsgemäß verwendbare Emulgatoren sind beispielsweise
- Anlagerungsprodukte von 4 bis 100 Mol Ethylenoxid und/oder 1 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- lineare und verzweigte Fettsäuren mit 8 bis 30 C - Atomen und deren Na-, K-, Ammonium-, Ca-, Mg- und Zn - Salze.

Die Emulgatoren werden bevorzugt in Mengen von 0,1 bis 25 Gew.-%, insbesondere 0,1 bis 3 Gew.-%, bezogen auf die jeweilige gesamte Zusammensetzung, eingesetzt.

Bevorzugt sind nichtionogene Emulgatoren mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen. Nichtionogene Emulgatoren mit einem HLB-Wert von 10 bis 16 sind erfindungsgemäß besonders bevorzugt.

Handelt es sich bei den erfindungsgemäßen Mitteln um Haarfärbemittel, so enthalten diese weiterhin mindestens ein Oxidationsfarbstoffvorprodukt und/oder mindestens einen direktziehenden Farbstoff. Als Oxidationsfarbstoffvorprodukt eignen sich die bekannten Entwicklerkomponenten, die gegebenenfalls in Kombination mit mindestens einer Kupplerkomponente eingesetzt werden.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 11, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Die erfindungsgemäßen Mittel gemäß dieser Ausführungsform enthalten die direktziehenden Farbstoffe bevorzugt in einer Menge von 0,001 bis 20 Gew.-%, bezogen auf das gesamte Mittel.

Weiterhin können die erfindungsgemäßen Mittel auch in der Natur vorkommende Farbstoffe enthalten, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Es ist nicht erforderlich, dass die direktziehenden Farbstoffe jeweils einheitliche Verbindungen darstellen. Vielmehr können in den erfindungsgemäßen Mitteln, bedingt durch die Herstellungsverfahren für die einzelnen Farbstoffe, in untergeordneten Mengen noch weitere Komponenten enthalten sein, soweit diese nicht das Stylingergebnis nachteilig beeinflussen oder aus anderen Gründen, z.B. toxikologischen, ausgeschlossen werden müssen.

Die Mittel können neben den genannten Komponenten weiterhin alle für entsprechende kosmetische Mittel bekannten Wirk-, Zusatz- und Hilfsstoffe enthalten.

Weitere Wirk-, Hilfs- und Zusatzstoffe, sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit, vollsynthetische Hydrokolloide wie z.B. Polyvinylalkohol, und gegebenenfalls vernetzte Polyacrylate,
- Strukturanten wie Maleinsäure und Milchsäure,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Substanzen zur Einstellung des pH-Wertes, wie beispielsweise übliche Säuren, insbesondere Genusssäuren, und Basen,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie der eingesetzten Mengen dieser Komponenten wird ausdrücklich auf die dem Fachmann bekannten einschlägigen Handbücher verwiesen.

Die Formulierung der erfindungsgemäßen Mittel kann in allen für kosmetische Mittel üblichen Formen erfolgen, beispielsweise in Form von Lösungen, die als Gesichts- oder Haarwasser oder Pump- oder Aerosolspray auf die Haut oder das Haar aufgebracht werden können, in Form von Cremes, Emulsionen, Wachsen, Gelen oder auch tensidhaltigen schäumenden Lösungen oder anderen Zubereitungen, die für die Anwendung auf der Haut oder dem Haar geeignet sind.

Vorzugsweise handelt es sich jedoch bei den erfindungsgemäßen Mitteln um Mittel zur temporären Verformung keratinischer Fasern, d.h. um Stylingmittel. Bevorzugte Stylingmittel sind Stylinggele, Pumphaarsprays, Aerosolhaarspray, Pumphaarschäume und Aerosolhaarschäume.

Unter Haarschäumen werden dabei Zusammensetzungen verstanden, die bei der Entnahme aus einem geeigneten Behälter einen Schaum ausbilden. Es kann notwendig sein, den Mitteln Inhaltsstoffe zuzusetzen, die die Schaumbildung fördern oder einmal gebildeten Schaum stabilisieren. Insbesondere eignen sich dafür Tenside und/oder Emulgatoren, wie sie bereits oben beschrieben wurden. Vorzugsweise werden Tenside aus der Gruppe der kationischen Tenside eingesetzt.

Haarcremes und Haargele enthalten in der Regel Strukturanten und/oder verdickende Polymere, die dazu dienen, den Produkten die gewünschte Konsistenz zu verleihen. Strukturanten und/oder verdickende Polymere werden typischerweise in einer Menge von 0,1 bis 10 Gew.-%, bezogen auf das gesamte Produkt, eingesetzt. Mengen von 0,5 bis 5 Gew.-%, insbesondere 0,5 bis 3 Gew.-% sind bevorzugt. Da die erfindungsgemäß eingesetzte Polymerkombination jedoch selbstverdickende Eigenschaften aufweist, ist die Zugabe weiterer Strukturanten und/oder verdickender Polymere nicht zwingend erforderlich. Vorzugsweise enthalten die erfindungsgemäßen Mittel keine weiteren Strukturanten und/oder verdickender Polymere.

Sofern es sich bei den erfindungsgemäßen Mitteln um ein Aerosolprodukt handelt, enthält dieses zwingend ein Treibmittel.

Erfindungsgemäß geeignete Treibmittel sind beispielsweise N₂O, Dimethylether, CO₂, Luft und Alkane mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und deren Mischungen.

Bevorzugt werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Über das Mengenverhältnis von Treibmittel zu den übrigen Bestandteilen der Zubereitungen lassen sich bei gegebener Sprühvorrichtung die Größen der Aerosoltröpfchen bzw. der Schaumblasen und die jeweilige Größenverteilung einstellen.

Die Menge an eingesetztem Treibmittel variiert in Abhängigkeit von der konkreten Zusammensetzung des Mittels, der verwendeten Verpackung und der gewünschten Produktart, etwa Haarspray oder Haarschaum. Bei Verwendung herkömmlicher Sprühvorrichtungen enthalten Aerosolschaumprodukte das Treibmittel bevorzugt in Mengen von 1 bis 35 Gew.-%, bezogen auf das gesamte Produkt. Mengen von 2 bis 30 Gew.-%, insbesondere von 3 bis 15 Gew.-% sind besonders bevorzugt. Aerosolsprays enthalten generell größere Mengen an Treibmittel. Bevorzugt wird das Treibmittel in diesem Fall in einer Menge von 30 bis 98 Gew.-%, bezogen auf das gesamte Produkt, eingesetzt. Mengen von 40 bis 95 Gew.-%, insbesondere von 50 bis 95 Gew.-% sind besonders bevorzugt.

Die Aerosolprodukte lassen sich in üblicher Art und Weise herstellen. In der Regel werden alle Bestandteile des jeweiligen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

Ein zweiter Gegenstand der Erfindung ist daher ein Verfahren, bei dem das erfindungsgemäße, kosmetisches Mittel auf das Haar als Pumphaarspray, Aerosolhaarspray, Pumphaarschaum, Aerosolhaarschaum oder Stylinggel aufgetragen wird und gegebenenfalls mit den Handflächen und/oder den Fingern in das Haar eingearbeitet wird.

Die gewünschte Verformung der Haare kann dabei mit den Finger oder Händen sowie mit geeigneten, herkömmlichen Hilfsmitteln wie beispielsweise Kamm oder Bürste erfolgen.

Ein dritter Gegenstand der Erfindung ist die Verwendung der erfindungsgemäßen Mittel zur temporären Verformung keratinischer Fasern.

Die erfindungsgemäßen Mittel und Produkte, die diese Mittel enthalten, zeichnen sich insbesondere dadurch aus, dass sie behandeltem Haar einen sehr starken und feuchtebeständigen Frisurenhalt verleihen.

### Beispiele

Die nachfolgenden Beispiele sollen den Gegenstand der vorliegenden Erfindung erläutern ohne ihn in irgendeiner Weise zu beschränken.

Die folgenden Mengenangaben verstehen sich - soweit nichts anderes vermerkt ist - in Gewichtsprozent.

### Beispiel 1

Es wurden die erfindungsgemäßen Stylingmittel A bis F gemäß folgender Tabelle hergestellt.

| **Rohstoffe** | **A** | **B** | **C** | **D** | **E** | **F** |
|---|---|---|---|---|---|---|
| Triethanolamin, 99% | 1,625 | 1,625 | 1,625 | 1,625 | 1,625 | 1,625 |
| Luviflex Soft ¹ | 8,335 | 8,335 | 8,335 | 8,335 | 8,335 | 8,335 |
| Diaformer Z 632N ² | 8,33 | - | - | - | - | - |
| Diaformer Z-651N ³ | - | 8,33 | - | - | - | - |
| Diaformer Z-711N ⁴ | - | - | 6,25 | - | - | - |
| Diaformer Z-712N ⁵ | - | - | - | 6,25 | - | - |
| Diaformer Z-712W ⁶ | - | - | - | - | 11,90 | - |
| Diaformer Z-731N ⁷ | - | - | - | - | - | 6,25 |
| Wasser, vollentsalzt | ad 100 | ad 100 | ad 100 | ad 100 | ad 100 | ad 100 |

| | | | | | | |
|---|---|---|---|---|---|---|
| ¹ Copolymer aus Ethylacrylat und Methacrylsäure, 30% Dispersion in Wasser (INCI-Bezeichnung: Acrylates Copolymer) (BASF) ² Copolymerisat aus Stearylacrylat, Methacryloylethylaminoxid und einem oder mehreren Monomeren aus Acrylsäure, Methacrylsäure und deren einfache Ester (28-32 Gew.-% Festkörper in Ethanol; INCI-Bezeichnung: Acrylates/Stearyl Acrylate/Ethylamine Oxide Methacrylate Copolymer) (Clariant) ³ Copolymerisat aus Stearylacrylat, Laurylacrylat, Methacryloylethylaminoxid und einem oder mehreren Monomeren aus Acrylsäure, Methacrylsäure und deren einfache Ester (28-32 Gew.-% Festkörper in Ethanol/Wasser (6:1); INCI-Bezeichnung: Acrylates/Stearyl Acrylate/ Lauryl Acrylate/ Ethylamine Oxide Methacrylate Copolymer) (Clariant) ⁴ Copolymerisat aus Stearylacrylat, Laurylacrylat, Methacryloylethylaminoxid und einem oder mehreren Monomeren aus Acrylsäure, Methacrylsäure und deren einfache Ester (38-42 Gew.-% Festkörper in Ethanol/Wasser (5:1); INCI-Bezeichnung: Acrylates/Stearyl Acrylate/ Lauryl Acrylate/ Ethylamine Oxide Methacrylate Copolymer) (Clariant) ⁵ Copolymerisat aus Stearylacrylat, Laurylacrylat, Methacryloylethylaminoxid und einem oder mehreren Monomeren aus Acrylsäure, Methacrylsäure und deren einfache Ester (38-42 Gew.-% Festkörper in Ethanol/Wasser (5:1); INCI-Bezeichnung: Acrylates/Stearyl Acrylate/ Lauryl Acrylate/ Ethylamine Oxide Methacrylate Copolymer) (Clariant) ⁶ Copolymerisat aus Stearylacrylat, Laurylacrylat, Methacryloylethylaminoxid und einem oder mehreren Monomeren aus Acrylsäure, Methacrylsäure und deren einfache Ester (38-42 Gew.-% Festkörper in Wasser; INCI-Bezeichnung: Acrylates/Stearyl Acrylate/ Lauryl Acrylate/ Ethylamine Oxide Methacrylate Copolymer) (Clariant) ⁷ Copolymerisat aus Stearylacrylat, Laurylacrylat, Methacryloylethylaminoxid und einem oder mehreren Monomeren aus Acrylsäure, Methacrylsäure und deren einfache Ester (38-42 Gew.-% Festkörper in Ethanol/Wasser (5:1); INCI-Bezeichnung: Acrylates/Stearyl Acrylate/ Lauryl Acrylate/ Ethylamine Oxide Methacrylate Copolymer) (Clariant) | | | | | | |

Haarsträhnen (Fa. Kerling Internationale Haarfabrik GmbH, Art.-Nr. 826500 Klebetresse dicht; 0.9 g) wurden mit Natrium Laurethsulfat (12.5 Gew.-% in Wasser) gewaschen und mit Ethanol (50 Gew.-% in Wassser) bei 50°C behandelt. Anschließend wurden die Strähnen mit lauwarmem Wasser gründlich nachgespült und durchgekämmt. Überschüssiges Wasser wurde aus den Strähnen zwischen Zeigefinger und Daumen ausgestrichen und die Strähnen wurden über Nacht bei 45°C getrocknet. Anwendungenvorschriften für:
- Hairsprays:
   Die Strähne wird gleichmäßig aus einer Distanz von 10 cm auf jeder Seite für 2 s besprüht.
- Styling mousses:
   Die Mousse wird im Gewichtsverhältnis 1:2 zum Strähnengewicht eingesetzt und auf der Strähne mit den Fingern verteilt und eingearbeitet.
- Gel/Crème/Wachse:
   Das Gel wird im Gewichtsverhältnis 1:2 zum Strähnengewicht eingesetzt und auf der Strähne mit den Fingern verteilt und eingearbeitet.

Die präparierte Strähne wird auf einer Platte für 1 h bei 45°C getrocknet und anschließend von der Platte vorsichtig mit einer Nadel, die zuvor zwischen Strähne und Platte plaziert worden war, entfernt.

Die mit den Stylinggelen A bis F präparierten Strähnen wurden mittels Clips nebeneinander und parallel an einer Stange befestigt. Die Stange wird so gedreht, dass alle Strähnen horizontal ausgerichtet sind. Anschließend wird die Stange um 90° gedreht, und die jetzt vertikal ausgerichteten Strähnen werden für 5 s in eine Box, die mit auf Raumtemperatur temperiertem Wasser beziehungsweise Salzwasser (als Simulation von Schweiß) gefüllt ist, eingetaucht. Anschließend wird die Stange wieder um 90° zurückgedreht und die Auslenkung der Haarsträhnen gegenüber dem Ausgangswert bestimmt.
Alle erfindungsgemäßen Beispiele zeichnen sich durch eine exzellente Formstabilität nach Eintauchen in Wasser und durch eine sehr gute Formstabilität nach Eintauchen in Salzwasser aus (siehe Tabelle 1).

**Tabelle 1. Formstabilität im Eintauchtest**

| **Styling gel** | **Auslenkung nach Wasser¹** | **Auslenkung nach Salzwasser¹** |
|---|---|---|
| A | 98% | 92% |
| B | 104% | 96% |
| C | 109% | 93% |
| D | 105% | 93% |
| E | 101% | 86% |
| F | 97% | 87% |

| | | |
|---|---|---|
| ¹ Ein Wert von 100% entspricht keiner Auslenkung, d. h. komplette Formstabiltität, ein Wert kleiner 100% entspricht einer entsprechenden Auslenkung aus der Horizontallage und damit einem Verlust der Formstabilität. Abweichungen von +/- 5% liegen im Rahmen der Messgenauigkeit. | | |

### Beispiel 2

In einem Vergleichsexperiment wurden vorbehandelte Haarsträhnen mit dem Stylinggel der erfindungsgemäßen Zusammensetzung sowie mit zwei handelsüblichen Stylinggelen in jeweils gleicher Anwendungsmenge präpariert und getrocknet. Als handelsübliche Stylinggele wurden ein Gel auf Polyvinylpyrrolidinon-Basis sowie ein Gel auf Vinylpyrrolidinon/Vinylacetamid/ Vinylimidazol-Copolymer-Basis eingesetzt. Die so präparierten Haarsträhnen wurden mittels Clips nebeneinander und parallel an einer Stange befestigt.
i) Eine horizontale Aufhängung der Strähnen führte zu keiner Abknickung der mit erfindungsgemäßen wie handelsüblichen Mitteln präparierten Strähnen, wodurch die äquivalente Formstabilität der erfindungsgemäßen Zusammensetzung gegenüber handelsüblichen Stylinggelen nachgewiesen ist.
ii) In einem weiteren Schritt wurden die präparierten Strähnen vertikal in ein auf Raumtemperatur temperiertes Wasserbad gehängt. Bereits nach 1 Minute im Wasserbad konnte ein deutlicher Verlust der Formstabilität bei den mit handelsüblichen Stylinggelen präparierten Strähnen beobachtet werden, während die mit der erfindungsgemäßen Zusammensetzung präparierten Strähne keine Stabilitätsverluste aufwies.
iii) Nach 4 Minuten im Wasserbad waren die handelsüblichen Stylinggele soweit gelöst, dass keine Formstabilität der präparierten Strähnen mehr gegeben war, während die erfindungsgemäß präparierte Strähne weiterhin formstabil blieb.
iv) Nach 5 Minuten schließlich wurden die Strähnen aus dem Wasserbad genommen und erneut horizontal aufgehängt. Die mit handelsüblichen Stylinggelen präparierten Strähnen hingen ohne Halt und Form herab, während die erfindungsgemäß präparierte Haarsträhne nach wie vor weitestgehend ihre Formstabilität erhalten hatte und nahezu waagrecht ausgerichtet verblieb.

## Patentansprüche

1. Kosmetisches Mittel, enthaltend in einem kosmetisch akzeptablen Träger
a) mindestens ein Copolymer A, gebildet aus
- mindestens einem Monomer A1 ausgewählt aus Acrylsäure, Methacrylsäure, Acrylsäurealkylestern und Methacrylsäurealkylestern, und
- mindestens einem Monomer A2 ausgewählt aus (Meth)acryloylalkylbetainen der Formel A2-I 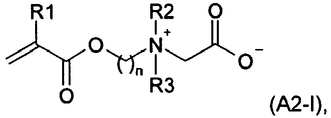 und (Meth)acryloylalkylaminoxiden der Formel A2-II 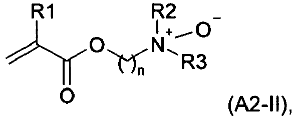 wobei in Formel A2-I und in Formel A2-II
R1 für H oder CH₃,
R2 und R3 jeweils unabhängig voneinander für gegebenenfalls verzweigtes C₁-C₁₀-Alkyl und
n für eine ganze Zahl von 1 bis 20 steht,
b) und mindestens ein von Copolymer A unterschiedliches Metharcylsäure/Ethylacrylat-Copolmyer als filmbildendes und/oder festigendes anionisches Polymer B.

2. Mittel nach Anspruch 1, **dadurch gekennzeichnet, dass** Monomer A1 ausgewählt ist aus Acrylsäure, Methacrylsäure, Acrylsäure-C₁-C₂₀-alkylestern und Methacrylsäure-C₁-C₂₀-alkylestern.

3. Mittel nach einem der Ansprüche 1 oder 2, **dadurch gekennzeichnet, dass** Monomer A2 ausgewählt ist aus (Meth)acryloylalkylbetainen der Formel A2-I und (Meth)acryloylalkylaminoxiden der Formel A2-II, wobei R2 und R3 jeweils unabhängig voneinander für Methyl, Ethyl, n-Propyl, *i*-Propyl, *n*-Butyl, *i*-Butyl oder *t*-Butyl stehen.

4. Mittel nach einem der Ansprüche 1 bis 3, **dadurch gekennzeichnet, dass** Monomer A2 ausgewählt ist aus (Meth)acryloylalkylbetainen der Formel A2-I und (Meth)acryloylalkylaminoxiden der Formel A2-II, wobei n jeweils für eine ganze Zahl von 1 bis 5, vorzugsweise für eine ganze Zahl von 1 bis 3 und besonders bevorzugt für 2 steht.

5. Mittel nach einem der Ansprüche 1 bis 4, **dadurch gekennzeichnet, dass** Monomer A2 ausgewählt ist aus Methacryloylalkylbetainen der Formel A2-I und Methacryloylalkylaminoxiden der Formel A2-II, wobei R1 jeweils für CH₃ steht.

6. Mittel nach Anspruch 1, **dadurch gekennzeichnet, dass** das Copolymer A gebildet ist aus
- mindestens einem Monomer A1 ausgewählt aus Acrylsäure, Methacrylsäure, Acrylsäuremethylester, Methacrylsäuremethylester, Acrylsäureethylester, Methacrylsäureethylester, Acrylsäurepropylester, Methacrylsäurepropylester, Acrylsäureisopropylester und Methacrylsäureisopropylester, und
- mindestens einem Methacryloylethylbetain der Formel A2-I als Monomer A2.

7. Mittel nach Anspruch 1, **dadurch gekennzeichnet, dass** das Copolymer A gebildet ist aus
- mindestens zwei Monomeren A1, wobei das erste Monomer ausgewählt ist aus Acrylsäure, Methacrylsäure, Acrylsäuremethylester, Methacrylsäuremethylester, Acrylsäureethylester, Methacrylsäureethylester, Acrylsäurepropylester, Methacrylsäurepropylester, Acrylsäureisopropylester und Methacrylsäureisopropylester, und das zweite Monomer ausgewählt ist aus Acrylsäurestearylester und Methacrylsäurestearylester, und
- mindestens einem Methacryloylethylaminoxid der Formel A2-II als Monomer A2.

8. Mittel nach Anspruch 1, **dadurch gekennzeichnet, dass** das Copolymer A gebildet ist aus
- mindestens drei Monomeren A1, wobei das erste Monomer ausgewählt ist aus Acrylsäure, Methacrylsäure, Acrylsäuremethylester, Methacrylsäuremethylester, Acrylsäureethylester, Methacrylsäureethylester, Acrylsäurepropylester, Methacrylsäurepropylester, Acrylsäureisopropylester und Methacrylsäureisopropylester, das zweite Monomer ausgewählt ist aus Acrylsäurelaurylester und Methacrylsäurelaurylester, und das dritte Monomer ausgewählt ist aus Acrylsäurestearylester und Methacrylsäurestearylester, und
- mindestens einem Methacryloylethylaminoxid der Formel A2-II als Monomer A2.

9. Mittel nach einem der Ansprüche 1 bis 8, **dadurch gekennzeichnet, dass** es das Copolymer A in einer Menge von 0,01 bis 20 Gew.%, vorzugsweise 0,1 bis 15 Gew.-%, besonders bevorzugt 1,0 bis 12 Gew.%, bezogen auf das gesamte Mittel, enthält.

10. Mittel nach einem der Ansprüche 1 bis 9, **dadurch gekennzeichnet, dass** es das filmbildende und/oder festigende anionische Polymer B in einer Menge von 0,01 bis 20 Gew.-%, vorzugsweise 0,1 bis 15 Gew.-%, besonders bevorzugt 1,0 bis 10 Gew.%, bezogen auf das gesamte Mittel, enthält.

11. Mittel nach einem der Ansprüche 1 bis 10, **dadurch gekennzeichnet, dass** es das Copolymer A und das filmbildende und/oder festigende anionische Polymer B in einem Gewichtsverhältnis von 1:20 bis 20:1, vorzugsweise von 1:10 bis 10:1, besonders bevorzugt von 1:5 bis 5:1, ganz besonders bevorzugt 1:3 bis 3:1 enthält.

12. Verfahren, bei dem ein kosmetisches Mittel nach einem der Ansprüche 1 bis 11 auf das Haar als Pumphaarspray, Aerosolhaarspray, Pumphaarschaum, Aerosolhaarschaum oder Stylinggel aufgetragen wird und gegebenenfalls mit den Handflächen und/oder den Fingern in das Haar eingearbeitet wird.

13. Verwendung eines Mittels gemäß wenigstens eines der Ansprüche 1 bis 11 zur temporären Verformung von Haaren.

## Claims

1. A cosmetic agent containing in a cosmetically acceptable carrier
a) at least one copolymer A, formed by
- at least one monomer A1 selected from acrylic acid, methacrylic acid, acrylic acid alkyl esters and methacrylic acid alkyl esters, and
- at least one monomer A2 selected from (meth)acryloyl alkyl betaines of formula A2-I 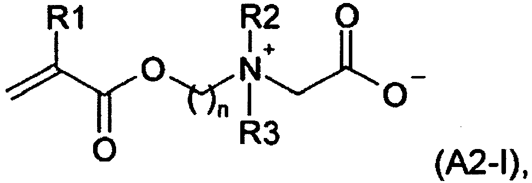 and (meth)acryloyl alkylamine oxides of formula A2-II 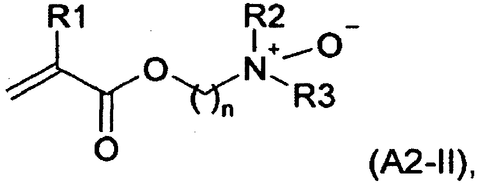 wherein, in formula A2-I and in formula A2-II,
R1 represents H or CH₃,
R2 and R3 each represent, independently of one another, optionally branched C₁-C₁₀ alkyl, and
n represents an integer from 1 to 20,
b) and at least one methacrylic acid/ethyl acrylate copolymer that is different from copolymer A as a film-forming and/or strengthening anionic polymer B.

2. The agent according to claim 1, **characterized in that** monomer A1 is selected from acrylic acid, methacrylic acid, acrylic acid C₁-C₂₀ alkyl esters and methacrylic acid C₁-C₂₀ alkyl esters.

3. The agent according to claim 1 or 2, **characterized in that** monomer A2 is selected from (meth)acryloyl alkyl betaines of formula A2-I and (meth)acryloyl alkylamine oxides of formula A2-II, where R2 and R3 each represent, independently of one another, methyl, ethyl, n-propyl, i-propyl, n-butyl, i-butyl or t-butyl.

4. The agent according to one of claims 1 to 3, **characterized in that** monomer A2 is selected from (meth)acryloyl alkyl betaines of formula A2-I and (meth)acryloyl alkylamine oxides of formula A2-II, where n in each case represents an integer from 1 to 5, preferably an integer from 1 to 3, and particularly preferably 2.

5. The agent according to one of claims 1 to 4, **characterized in that** monomer A2 is selected from methacryloyl alkyl betaines of formula A2-I and methacryloyl alkylamine oxides of formula A2-II, where R1 in each case represents CH₃.

6. The agent according to claim 1, **characterized in that** the copolymer A is formed by
- at least one monomer A1 selected from acrylic acid, methacrylic acid, acrylic acid methyl ester, methacrylic acid methyl ester, acrylic acid ethyl ester, methacrylic acid ethyl ester, acrylic acid propyl ester, methacrylic acid propyl ester, acrylic acid isopropyl ester and methacrylic acid isopropyl ester, and
- at least one methacryloyl ethyl betaine of formula A2-I as monomer A2.

7. The agent according to claim 1, **characterized in that** the copolymer A is formed by
- at least two monomers A1, the first monomer being selected from acrylic acid, methacrylic acid, acrylic acid methyl ester, methacrylic acid methyl ester, acrylic acid ethyl ester, methacrylic acid ethyl ester, acrylic acid propyl ester, methacrylic acid propyl ester, acrylic acid isopropyl ester and methacrylic acid isopropyl ester, and the second monomer being selected from acrylic acid stearyl ester and methacrylic acid stearyl ester, and
- at least one methacryloyl ethylamine oxide of formula A2-II as monomer A2.

8. The agent according to claim 1, **characterized in that** the copolymer A is formed by
- at least three monomers A1, the first monomer being selected from acrylic acid, methacrylic acid, acrylic acid methyl ester, methacrylic acid methyl ester, acrylic acid ethyl ester, methacrylic acid ethyl ester, acrylic acid propyl ester, methacrylic acid propyl ester, acrylic acid isopropyl ester and methacrylic acid isopropyl ester, the second monomer being selected from acrylic acid lauryl ester and methacrylic acid lauryl ester, and the third monomer being selected from acrylic acid stearyl ester and methacrylic acid stearyl ester, and
- at least one methacryloyl ethylamine oxide of formula A2-II as monomer A2.

9. The agent according to one of claims 1 to 8, **characterized in that** it contains the copolymer A in an amount of 0.01 to 20 wt.%, preferably 0.1 to 15 wt.%, particularly preferably 1.0 to 12 wt.%, based on the total agent.

10. The agent according to one of claims 1 to 9, **characterized in that** it contains the film-forming and/or strengthening anionic polymer B in an amount of 0.01 to 20 wt.%, preferably 0.1 to 15 wt.%, particularly preferably 1.0 to 10 wt.%, based on the total agent.

11. The agent according to one of claims 1 to 10, **characterized in that** it contains the copolymer A and the film-forming and/or strengthening anionic polymer B in a weight ratio of 1:20 to 20:1, preferably 1:10 to 10:1, particularly preferably 1:5 to 5:1, very particularly preferably 1:3 to 3:1.

12. A method in which a cosmetic agent according to one of claims 1 to 11 is applied to the hair as a pump hairspray, aerosol hairspray, pump hair foam, aerosol hair foam or styling gel and, if necessary, is worked into the hair using the palms of the hands and/or the fingers.

13. The use of an agent according to at least one of claims 1 to 11 for temporarily shaping hair.

## Revendications

1. Agent cosmétique contenant, dans un support cosmétiquement acceptable,
a) au moins un copolymère A constitué de
- au moins un monomère A1 choisi parmi l'acide acrylique, l'acide méthacrylique, des alkylesters d'acide acrylique et des alkylesters d'acide méthacrylique, et
- au moins un monomère A2 choisi parmi des (méth)acryloylalkylbétaïnes de formule A2-I 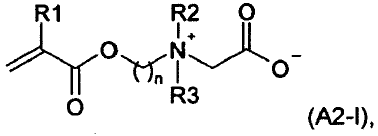 et parmi des (méth)acryloylalkylamines oxydes de formule A2-II 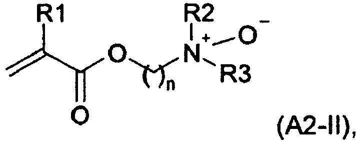 sachant que, dans la formule A2-I et la formule A2-II,
R1 représente H ou CH₃,
R2 et R3 représentent indépendamment l'un de l'autre un alkyle en C₁-C₁₀ ramifié le cas échéant et
n représente un nombre entier de 1 à 20,
b) et au moins un copolymère acide méthacrylique/acrylate d'éthyle différent du copolymère A comme polymère B anionique filmogène et/ou fixant.

2. Agent selon la revendication 1 **caractérisé en ce que** le monomère A1 est choisi parmi l'acide acrylique, l'acide méthacrylique, des alkylesters en C₁-C₂₀ d'acide acrylique et des alkylesters en C₁-C₂₀ d'acide méthacrylique.

3. Agent selon une des revendications 1 ou 2 **caractérisé en ce que** le monomère A2 est choisi parmi des (méth)acryloylalkylbétaïnes de formule A2-I et des amines oxydes de (méth)acryloylalkyle de formule A2-II, R2 et R3 représentant indépendamment l'un de l'autre le méthyle, l'éthyle, le n-propyle, l'i-propyle, le n-butyle, l'i-butyle ou le t-butyle respectivement.

4. Agent selon l'une quelconque des revendications 1 à 3 **caractérisé en ce que** le monomère A2 est choisi parmi des (méth)acryloylalkylbétaïnes de formule A2-I et des amines oxydes de (méth)acryloylalkyle de formule A2-II, n représentant respectivement un nombre entier de 1 à 5, de préférence un nombre entier de 1 à 3 et de manière particulièrement préférée 2.

5. Agent selon l'une quelconque des revendications 1 à 4 **caractérisé en ce que** le monomère A2 est choisi parmi des méthacryloylalkylbétaïnes de formule A2-I et des amines oxydes de méthacryloylalkyle de formule A2-II, R1 représentant respectivement CH₃.

6. Agent selon la revendication 1 **caractérisé en ce que** le copolymère A est constitué de
- au moins un monomère A1 choisi parmi l'acide acrylique, l'acide méthacrylique, un méthylester d'acide acrylique, un méthylester d'acide méthacrylique, un éthylester d'acide acrylique, un éthylester d'acide méthacrylique, un propylester d'acide acrylique, un propylester d'acide méthacrylique, un isopropylester d'acide acrylique et un isopropylester d'acide méthacrylique, et
- au moins une méthacryloyléthylbétaïne de formule A2-I comme monomère A2.

7. Agent selon la revendication 1 **caractérisé en ce que** le copolymère A est constitué de
- au moins deux monomères A1, le première monomère étant choisi parmi l'acide acrylique, l'acide méthacrylique, un méthylester d'acide acrylique, un méthylester d'acide méthacrylique, un éthylester d'acide acrylique, un éthylester d'acide méthacrylique, un propylester d'acide acrylique, un propylester d'acide méthacrylique, un isopropylester d'acide acrylique et un isopropylester d'acide méthacrylique, et le deuxième monomère étant choisi parmi un stéarylester d'acide acrylique et un stéarylester d'acide méthacrylique, et
- au moins un amine oxyde de méthacryloyléthyle de formule A2-II comme monomère A2.

8. Agent selon la revendication 1 **caractérisé en ce que** le copolymère A est constitué de
- au moins trois monomères A1, le première monomère étant choisi parmi l'acide acrylique, l'acide méthacrylique, un méthylester d'acide acrylique, un méthylester d'acide méthacrylique, un éthylester d'acide acrylique, un éthylester d'acide méthacrylique, un propylester d'acide acrylique, un propylester d'acide méthacrylique, un isopropylester d'acide acrylique et un isopropylester d'acide méthacrylique, le deuxième monomère étant choisi parmi un laurylester d'acide acrylique et un laurylester d'acide méthacrylique, et le troisième monomère étant choisi parmi un stéarylester d'acide acrylique et un stéarylester d'acide méthacrylique, et
- au moins un amine oxyde de méthacryloyléthyle de formule A2-II comme monomère A2.

9. Agent selon l'une quelconque des revendications 1 à 8 **caractérisé en ce qu'**il contient le copolymère A dans une quantité de 0,01 à 20 % en poids, de préférence de 0,1 à 15 % en poids, de manière particulièrement préférée de 1,0 à 12 % en poids, par rapport à l'agent total.

10. Agent selon l'une quelconque des revendications 1 à 9 **caractérisé en ce qu'**il contient le polymère B anionique filmogène et/ou fixant dans une quantité de 0,01 à 20 % en poids, de préférence de 0,1 à 15 % en poids, de manière particulièrement préférée de 1,0 à 10 % en poids, par rapport à l'agent total.

11. Agent selon l'une quelconque des revendications 1 à 10 **caractérisé en ce qu'**il contient le copolymère A et le polymère B anionique filmogène et/ou fixant dans un rapport pondéral de 1 :20 à 20 :1, de préférence de 1 :10 à 10 :1, de manière particulièrement préférée de 1 :5 à 5 :1, de manière tout particulièrement préférée de 1 :3 à 3 :1.

12. Procédé dans lequel un agent cosmétique est appliqué selon l'une quelconque des revendications 1 à 11 sur le cheveu sous forme de spray coiffant en pompe, spray coiffant en aérosol, mousse coiffante en pompe, mousse coiffante en aérosol ou gel coiffant et qu'on le fait éventuellement pénétrer dans le cheveu avec les paumes des mains et/ou les doigts.

13. Utilisation d'un agent selon au moins l'une quelconque des revendications 1 à 11 pour la mise en forme temporaire des cheveux.
